# EUROPEAN PATENT SPECIFICATION

(11) **EP 0 639 373 B1**
(45) Date of publication and mention of the grant of the patent: **02.12.1998**
(21) Application number: 94112931.4
(22) Date of filing: 18.08.1994
(51) Int. Cl.: A61K 9/127

(54) **Preparations for the external application of antiseptic agents and/or agents promoting the healing of wounds**
Mittel zur äusserlichen Anwendung von Antiseptika und/oder wundheilungsfördernder Stoffe
Préparations pour l'application externe d'agents antiseptiques et/ou d'agents favorisant la cicatrisation

(30) Priority: 20.08.1993 DE 9312509 U
(43) Date of publication of application: 22.02.1995
(73) Proprietor: Euroceltique S.A., Luxembourg (LU)
(72) Inventor: Rückert, Dieter, c/o Weinman, D-72760 Reutlingen (DE); Gümbel, Hermann, Dr., D-63303 Dreieich (DE); Fleischer, Wolfgang, Dr., D-55218 Ingelheim (DE); Reimer, Karen, Dr., D-65551 Limburg (DE); Winkler, Horst, Dr., D-65550 Limburg (DE)
(74) Representative: Maiwald, Walter, Dr. Dipl.-Chem.

(56) References cited:
- EP-A- 0 509 338
- EP-A- 0 613 685
- WO-A-90/11781
- US-A- 5 128 139
- DATABASE WPI Week 9038, Derwent Publications Ltd., London, GB; AN 90-287819 & JP-A-2 204 413 (YG NONOGAWA SHOJI) 14 August 1990
- DATABASE WPI Week 8827, Derwent Publications Ltd., London, GB; AN 88-188027 & JP-A-63 126 820 (SHISEIDO KK) 30 May 1988

## Description

The invention concerns preparations for the external application of agents with antiseptic and/or wound healing promoting properties. The preparations are specifically applied to wounds, skin, mucous membranes and mucosa-like unkeratinized epithelial tissues of humans and animals.

A plurality of different antibiotic and antiseptic agents are known for the topical treatment of infectious maladies. A decisive disadvantage of antibiotic agents is that the infecting bacteria show primary resistances, and can acquire secondary resistances, against these agents. Further, antibiotics quite often lead to patient sensibilisation. The use of antiseptics such as povidone iodine, also known as polyvidone iodine or PVP iodine, i.e. the poly(1-vinyl-2-pyrrolidin-2-one)-iodine complex, can prevent resistances. Antiseptic agents are also much more rarely allergenic as compared to antibiotics.

In the scientific literature liposomes have quite often been disclosed as drug carriers. A non-exhaustive list comprises the following, more recent publications:
Hoekstra, H. J., Van Baare, J., Dutrieux, R.P.:
   Evaluation of topical therapy and wound healing.
   European Burn Association 5th Congress, Brighton, England, 1993
Neuhann, T., Sommer, G.:
   Erfahrungen mit Jod-Povidon zur Behandlung der Keratokonjunctivitis epidemica.
   Z. prakt. Augenh. 1 (1980), 65
   wm:pk:sf
Pleyer, U., Schmidt, K., Thiel, H. J. (eds.):
   Liposomes in Ophthalmology and Dermatology.
   Hippokrates Verlag Stuttgart, 1993
Prüfer, K., Sternberg, B.:
   Liposomen in der Medizin - Eine aktuelle Bestandsaufnahme.
   Z. ärzt. Fortbildung 88 (1994), 257-256
Rubas, W., Schreier, H.:
   Liposomen: Fortschritt in Herstellungs-Technologie und Therapie
   Pharmazie in unserer Zeit, 6 (1991) 255-270
Schreier, H., Bouwstra, J.:
   Liposomes as topical drug carriers: dermal and transdermal drug delivery.
   (Submitted)
Shell, J.W.:
   Ophthalmic drug delivery systems.
   Surv. Opthalmol. 29 (19984), 117.

Further, the team of Hoekstra et al. in Beverwijk, Netherlands, has reported on animal experiments with silver sulphadiazine, a chemotherapeutic agent, encapsulated in liposomes and applied to experimental wounds. The results appear to show agent enrichment at the wound bottom and reduced silver resorption, compared with customary silver sulphadiazine ointments.

However, although a lot of attention has been paid for quite some time to liposomes as drug carriers, there appears to be no prior art relating to liposomes as carriers of antiseptic or wound healing promoting agents for external applications.

An object of the instant invention is to provide a well tolerated, easily applicable antiseptic or wound healing promoting preparation, which provides protracted release and protracted topical effect of the active agent.

According to the invention this technical object is attained in that the preparation comprises at least one antiseptic and/or wound healing promoting agent in the form of a liposome preparation, as defined in the independent claim.

The dependent claims define further advantageous embodiments of the invention.

The invention is premised on the surprising fact that liposomes are highly suited as carriers for antiseptic agents, especially for povidone iodine, and for agents promoting the healing of wounds.

The liposome preparations according to this invention permit protracted release of the agent or agents, and provide an extended and topical activity at the desired locus of action by interaction with cell surfaces.

Experiments and research carried out by the instant inventors appear to show that, even more unexpectedly, the preparations according to this invention do not only contain the active agent like povidone iodine encapsulated in liposomes. It seems that there is also some amount of agent which is not contained inside the liposomes. The preparations according to the invention often show a marked initial effect which is observed in addition to the slower, protracted release of the active agent from the liposomes. Without wishing to be bound to any theoretical explanation, it is presently assumed that in addition to active agent encapsulated inside the liposomes, some active agent is present outside of the liposomes, and probably losely bound to the outer surfaces of the liposomes. This could be due to association of active agent molecules with the liposomal membrane, or it could be due to active agent molecules forming a layer on the liposomal surface, which layer partly or even fully coats the liposome externally. The type and amount of this initial agent effect can e.g. be influenced by choice of the concentration parameters.

Liposome preparations according to this invention thus make it possible to achieve effects which cannot be provided by customary preparations such as solutions, ointments etc..

Preferred antiseptic agents comprise the well-known pharmaceutical substances providing fast effect, a broad range of activity, low systemic toxicity and good tissue compatibility. They can e.g. be selected from the group comprising iodine and iodine complexes. A specifically preferred antiseptic agent is povidone iodine.

Preferred agents promoting the healing of wounds comprise substances which have been described in the literature for such application. Preferred such agents include substances known to promote epithelisation. These include vitamins, specifically from the vitamin B group, allantoin, some azulenes etc..

In preferred embodiments, the liposome preparations containing antiseptic and/or wound healing promoting agents can comprise further agents such as anaesthetic agents. Inventive preparations can also contain customary further agents, including adjuvants and additives, conserving agents or consistency forming agents such as viscosity adjusting additives, emulgators etc..

The amphiphilic substances generally known in prior art to form liposome membranes can be employed in the context of the invention as long as they are pharmaceutically acceptable for the intended application. Presently, liposome forming systems comprising lecithine are preferred. Such systems can comprise hydrogenated soy bean lecithine besides cholesterol and disodium succinate-hexahydrate; it is presently specificially preferred to use hydrogenated soy bean lecithine as the sole membrane forming agent.

The known prior art methods for forming liposome structures can generally be used in the context of the invention. Broadly, these methods comprise mechanical agitation of a suitable mixture containg the membrane forming substance and water or an aqueous solution. Filtration through suitable membranes is preferred in forming a substantially uniform liposome size.

The size of the liposomes can vary over a broad range, generally from about 20 to about 20,000 nm. Liposomes with diameters between 50 and 4,000 nm are preferred and liposomes of approximately 1,000 nm diameter are presently most preferred.

One presently preferred field of application is in ophthalmology, e.g. in the treatment of bacterial and viral keratoconjunctivitis, and the pre-operative antiseptic prophylaxis.

A presently highly preferred use of the inventive liposome preparations is in the local treatment of infections of the frontal part of the eye, especially when the liposome preparations contain povidone iodine. Also in this indication, the inventive antiseptic preparations, especially those containing PVP iodine, have the great advantage of not causing resistances and lead to much less allergic reactions, while permitting a very cost-efficient therapy with a broad spectrum of effect. A povidone iodine liposome preparation according to this invention is e.g. effective against adenovirus, the most frequent cause of viral conjunctivitis. This effect is not provided by antibiotic agents. Further, a liposome preparation of a microbicidal agent such as povidone iodine provides protracted release of the agent from liposomes located in the frontal part of the eye. This leads to extended effect of the antimicrobial substance, and thus less frequent application, as compared with the customary antiseptic eyedrop preparations.

Preparations according to this invention can take a variety of forms, including solutions, dispersions, lotions, creams, ointments and gels.

Generally, the amout of active agents in an inventive preparation will be determined by the desired effect on the one hand and the carrying capacity of the liposome preparation for the agent on the other hand.

Broadly, a solution or dispersion of active agent in an inventive liposome preparation can range between the lower limit of effectiveness of the agent and the solubility or dispersability limit of the agent in the respective solvent or dispersant.

Similar considerations broadly limit the amout of agent in lotions, creams, ointments or gels, or any other such preparation.

More specifically, for an antiseptic such as povidone iodine, a solution or dispersion in an inventive liposome preparation can contain between 0.1 and 10 g of agent in 100 g of preparation. Such a preparation will then typically contain between 1 and 5 g of liposome membrane forming substance esepcially lecithine per 100 g of preparation.

In a lotion, which can be a hydrophilic or a lipophilic lotion, a typical range of active agent will be between 0.5 and 10 g agent, and between 3 and 8 g, preferrably about 5 g of liposome membrane forming agent such as hydrogenated soy bean lecithine, per 100 g of lotion. In the case of a hydrophilic lotion, electrolyte solution will often be used in preparing the liposome containing lotion. A lipophilic lotion will often be made from agent, membrane forming substance and lipophilic formation agents such as medium chain length triglycerides etc..

A hydrophilic cream comprising an inventive liposome preparation will generally comprise between 0.1 and 10 g agent, such as povidone iodine, together with between about 1 and 10 g membrane forming substance and further typical O/W cream forming additives, per 100 g of cream.

A comparable amphiphilic cream according to the invention will have similar contents of agent and membrane forming substance such as lecithine, and will have the typical further additives of an amphiphilic cream.

A hydrophilic ointment according to the invention can broadly comprise between 0.1 and 10 g agent and between 1 and 10 g liposome membrane forming substance such as lecithine, together with typical prior art ointment basis substances such as Macrogol (TM) and water, in 100 g of ointment.

A non-alcoholic hydrogel according to the invention could broadly comprise between 1 and 5 g agent such as povidone iodine, approximately 2 g lecithine and gel forming substances such as Carbopol (TM), with pH-adjusting agent and water to form 100 g of hydrogel.

More specific formulations are notable from the embodiment example.

The features and advantages of this invention will become notable in more detail from the ensuing description of preferred embodiments. In these embodiments which include a best mode, povidone iodine is exemplified as an antiseptic agent. This should, however, not be construed as a restriction of this invention to antiseptic agents or, among antiseptic agents, to povidone iodine, although such preparations are specifically preferred.

One preferred method for producing the invention's liposomes can generally be described as follows:
The lipid membrane forming components, e.g. lecithine, are dissolved in a suitable solvent such as chloroform or a 2:1 mixture of methanol and chloroform and are filtered under sterile conditions. Then, a lipid film is produced on a sterile high surface substrate, such as glass beads, by controlled evaporation of the solvent. In some cases, it can be quite sufficient to form the film on the inner surface of the vessel used in evaporating the solvent, without using a specific substrate to increase the surface.

An aqueous system is prepared from electrolyte components and the (one or more) active agents to be incorporated in the liposome preparation. Such an aqueous system can e.g. comprise 10 mmol/l sodium hydrogen phosphate and 0.9 % sodium chloride, at ph 7.4; the aqueous system will further comprise at least the desired amount of the active agent, which in the embodiment examples is povidone iodide. Often, the aqueous system will comprise an excess amount of agent or agents.

The liposomes are generally formed by agitating said aqueous system in the presence of said film formed by the lipid components. At this stage, further additives can be added to improve liposome formation; e.g. sodium cholate can be added. Liposome formation can also be influenced by mechanical action such as pressure filtration through e.g. polycarbonate membranes, or centrifuging. Generally, the raw liposome dispersion will be washed, e.g. with electrolyte solution as used in preparing the above-described solution of the active agent.

When liposomes with the required size distribution have been obtained and washed, they can be redispersed in an electrolyte solution as already described, often also comprising sugars such as saccharose or a suitable sugar substitute. The dispersion can be freeze-dried, and it can be lyophilysed. It can, prior to use, be reconstituted by addition of water and suitable mechanical agitation at the transition temperature of the lipid component, which for hydrogenated soy bean lecithine is e.g. 55°C.

In the following Examples, hydrogenated soy bean lecithine (EPIKURON (TM) 200 SH obtainable from Lukas Meyer, Germany or PHOSPOLIPON (TM) 90 H obtainable from Nattermann Phospholipid GmbH, Germany) was used. However, other pharmaceutically acceptable liposome membrane forming substances can be used instead, and the person skilled in the art will find it easy to select suitable alternative liposome forming systems from what is described in prior art.

### Embodiment Example I

In a 1000 ml glass flask, provided with glass beads for increased surface, 51.9 mg cholesterol and 213 mg hydrogenated soy bean lecithine were dissolved in a sufficient amount of a mixture of methanol and chloroform in a 2:1 ratio. The solvent was then evaporated under a vacuum until a film was formed on the inner surface of the flask and on the glass beads.

2.4 g PVP iodine (containing about 10% available iodine) were separately dissolved in 12 ml water.

Again in a separate vessel, 8.77 g sodium chloride and 1.78 g Na₂HPO₄·2H₂O were dissolved in 400 ml water. Further water was added up to a total volume of 980 ml, and then, approximately 12 ml 1N hydrochloric acid were added to adjust pH to 7.4. This solution was then topped up with water to exactly 1000 ml.

In a fourth vessel, 900 mg saccharose and 57 mg disodium succinate were dissolved in 12 ml water.

The PVP iodine solution was then added to the lipid film in the flask and the mixture was shaken until the film dissolved. This produced liposome formation from the hydrated lipids in the flask. The product was centrifuged and the supernatant liquid was discarded. The saccharose solution was added ad 12 ml and the product was again centrifuged. Afterwards the supernatant liquid was again discarded. At this stage, a further washing step, using the saccharose solution or the sodium chloride buffer solution could be used.

After the last centrifugation step and discarding of the supernatant, sodium chloride buffer solution was added ad 12 ml, and the liposomes were homogenously distributed therein. The product was then distributed into vials each containing 2 ml liposome dispersion, and the vials were then subjected to a freeze-drying step.

After the freeze-drying, each vial comprised about 40 mg solids.

The method of Embodiment Example I has a minor disadvantage in that the PVP iodine solution used, due to the high percentage of solids, is rather viscous and thus more difficult to handle.

### Embodiment Example II

In a 2000 ml flask provided with glass beads to increase surface, 173 mg hydrogenated soy bean lecithine and 90 mg disodium succinate were dissolved in approximately 60 ml of a methanol/chloroform mix in a 2:1 ratio. The solvent was removed under vacuum until a film was formed.

4 g PVP iodine (10% available iodine) were dissolved in 40 ml of the sodium chloride buffer solution described in Embodiment Example I, and were added to the lipid film in the flask. The flask was then shaken until the film dissolved and liposomes were formed.

The product was centrifuged and the supernatant liquid was discarded.

To the thus produced liposome pellet, further sodium chloride buffer solution was added ad 40 ml, and the centrifuging step was repeated. The supernatant was again discarded. At this stage, this washing step could be repeated where necessary.

After the final centrifuging and decanting step, sodium chloride buffer solution was again added to the precipitated liposomes ad 40 ml. The homogenous dispersion was then distributed into vials, each vial containing about 2 ml liposome dispersion, and the vials were then subjected to a freeze-drying step. This produced approximately 200 mg freeze-dried solids per vial.

From the freeze-dried solids of Examples I and II, further preparations were made as described in subsequent Embodiment Examples and Test Reports.

Like that of Embodiment Example I, the above-described method uses a hydrating step after film formation in the presence of organic solvents and aims at inclusion rates of 5 bis 15%. These methods generally produce rather large and often multilamellar liposomes.

The above-described methods can be modified by a high pressure filtering step through a suitable membrane such as a polycarbonate membrane after the raw liposomes have been formed or after any of the subsequent washing steps or directly by using high pressure homogenisation. This produces much smaller, unilamellar liposomes at increased amounts of encapsulated agent.

Instead of high pressure homogenisation, other prior art methods known to provide small uniform sized liposomes can be employed.

### Embodiment Example III

A hydrophilic (O/W) cream was prepared from 10 g hydrogenated soy bean lecithine/PVP iodine liposomes as described in Embodiment Example II; these were mixed with 4 g Polysorbate 40 (TM), 8 g cetylstearyl alcohol, 8 g glycerol, 24 g white vaseline, and water ad 100 g.

### Embodiment Example IV

An amphiphilic cream was prepared from 10 g hydrogenated soy bean lecithine/povidone iodine liposomes as described in Embodiment Example II; 7.5 g medium chain length tryglyceride, 7 g polyoxyethyleneglycerol monostearate, 6 g cetylstearyl alcohol, 8 g propylene glycol, 25 g white vaseline, and water ad 100 g.

### Embodiment Example V

A hydrophilic ointment which can be rinsed off with water was prepared using 10 g of liposomal PVP iodine as described in Embodiment Example II, 55 g Macrogol 400 (TM), 25 g Macrogol 4000 (TM), and water ad 100 g.

### Embodiment Example VI

A hydrogel was prepared from 4 g liposomal PVP iodine as described in Embodiment Example II, 0.5 g Carbopol 980 NF (TM), sodium hydroxide ad pH 7, water ad 100 g.

Further modifications of the above-described embodiments are envisaged.

Thus, the creams of Embodiment Examples III and IV can have an additional content of an agent known to promote the healing of wounds, such as allantoin. Such an agent will be added in a pharmaceutically useful concentration, in the case of allantoin in the range of 0.1 to 0.5 g, per 100 g of cream.

The wound healing agent can be incorporated in the cream base, in which case it will largely be outside the liposomes. It can, however, be partly or mostly incorporated in the liposomes, in which case it will be added at a corresponding suitable stage of the liposome preparation method.

Similar alternatives are easily envisaged on the basis of the further Embodiment Examples.

It is also possible to prepare embodiments similar to the above described ones, which comprise an agent capable of promoting the healing of wounds instead of, and not in addition to, the antiseptic agent as e.g. povidone iodine disclosed in the above Embodiment Examples. Presently, it is however preferred to use a wound healing promoting agent (if at all) in addition to an antiseptic agent.

For application of the inventive preparations to a patient, known systems can be used, such as pneumatic pump applicators, two-chamber gas pressure packs etc..

In a pneumatic pump applicator, a bellows device is provided between an upstream and a downstream valve, both valves operating one way in the same direction. A supply of pharmaceutical preparation, such as an ointment or gel, is contained in a reservoir upstream of the valves- and -bellows device.

When compressing the bellows, the downstream valve opens and permits a dosed amount of preparation to leave the device for application. When the bellows is extended, this valve shuts and prevents reentry of the preparation. At the same time, the upstream valve opens and permits preparation from the reservoir to enter into the bellows, for release through the downstream valve upon the next compression step of the bellows.

The reservoir is sealed by a closure element which can move through the reservoir like a piston moves in a cylinder. By the stepwise emptying of the reservoir, this closure element is sucked into the reservoir, so that the remaining amount of pharmaceutical preparation in the reservoir is always sealed off, while at the same time the reservoir can be emptied.

Such a device is useful for pasty preparations, creams, ointments etc..

In a two-chamber gas pressure pack, the pharmaceutical preparation is contained in a bag of flexible plastics film material. Often, this is high pressure polyethylene.

The bag is contained inside a gas tight pressure vessel which further contains a supply of pressurizing gas, very often a compressed inert gas like nitrogen or air.

The plastic film bag has only one outlet, which is gas-tightly connected to the interior wall of the pressure vessel, surrounding a single opening thereof. The pressurized gas in the vessel tends to compress the bag, driving the pharmaceutical preparation inside the bag out through the opening of the bag and thus through the opening of the vessel. A valve and, in case, spray-head device is provided in the vessel mouth. Operating the valve releases a spray mist, a jet of liquid or a portion of flowable solid such as cream. Using such a system, solutions, emulsions, creams, oitments and gels can be dosed and applied.

Using inventive preparations efficiency and acceptability tests were then carried out, as follows:

### Test I

This was an in-vitro-test of the bactericidal effect provided by an inventive povidone iodine liposome preparation. The test was based on the quantitative suspension test as described in "Richtlinien der Deutschen Gesellschaft für Hygiene und Mikrobiologie", 1989. In this test, the bactericidal agent is used to kill staphylococcus aureus (ATCC 29213), a major problem in hospital hygiene.

The liposome preparation used was that of Embodiment Example I. At different contact times between 1 und 120 minutes, the minimum concentration of the preparation in water was determined which was capable of killing the staphilococci.

The results are shown in Table 1.

**TABLE I**

| Contact Time (Minutes) | Bactericidal Concentration |
|---|---|
| 1, 2, 3, 4 | ≥ 0.060 % |
| 5, 30, 60 | ≥ 0.015 % |
| 120 | ≥ 0.007 % |

The results show that at short contact times (between 1 and 4 minutes) the bactericidal concentration is as low as 0.06 % and that at long contact times (120 minutes) the bactericidal concentration can be as low as 0.007 %.

### Test II

The second test was a placebo-controlled clinical study of the local acceptability (at the eye) of an inventive povidone iodine liposome preparation. An eyedrop formulation was made using the liposomes of Embodiment Example II. It was tried on 15 male test persons. The inventive preparation was always used on one eye of the test person, with physiological sodium chloride solution added as a comparison to the respective other eye.

Specifically, each test person received one drop of PVP iodine liposome preparation in the right eye and one drop of physiological sodium chloride solution in the left eye, and this was twice repeated at hourly intervals. After 5, 30, 65, 95, 125 and 150 minutes as well as after 24 hours after the first application, symptoms were determined. These symptoms included hyperaemia, as measured with a slit/lamp microscope; burning; itching, and tear flow. Each symptom was measured according to a 4 point score with 0 corresponding to no symptom, 1 corresponding to a low degree, 2 corresponding to a medium degree and 3 corresponding to a strong degree of symptom appearance.

A sum score was calculated from the degree scores of all four symptoms and the 7 determination time points. The sum score could thus vary between 0 (= 0 times 0 times 0) and 84 (= 4 times 3 times 7).

The test persons were between 21 and 36 years old, with an average of 30 years of age. All test persons were healthy and not under medication during the test. Specifically, any illnesses of the eye and of the thyroid were excluded.

One test person was not evaluated for sum score since one control of symptoms after 150 minutes was missed.

The results are notable from Table II.

Overall, the sum score on both eyes was extremely low. It is surprising that on average, the sum score for the eyes treated with the povidone iodine liposome preparation was even lower than that for the eyes receiving physiological sodium chloride solution.

**TABLE II**

| Sum score | PVP-I-Liposomes | Phys. NaCl-Solution |
|---|---|---|
| | Number of test persons | |
| 0 | 11 | 6 |
| 1 | 3 | 6 |
| 2 | 0 | 2 |
| 3 - 84 | 0 | 0 |
| Average | 0.21 | 0.71 |
| Standard Deviation | 0.43 | 0.73 |
| Median | 0 | 1 |
| p-Value | 0,02 | |

11 test persons treated with the invention's povidone iodine liposome preparation showed no symptoms whatsoever. Three test persons had slight hyperaemia, one felt some very slight burning (this is the above-mentioned test person who could not be evaluated for sum score). On the contrary, only six test persons exhibited no symptoms after receiving physiological sodium chloride solution. Four test persons experienced burning, one of them at two subsequent time points. One test person experienced slight burning and itching of the left eye. A total of four test persons showed some hyperaemia.

## Claims

1. A pharmaceutical preparation for the external application of antiseptic agents and/or agents which promote the healing of wounds,
characterized in that the preparation contains at least one of said antiseptic agent, selected from the group comprising iodine and iodine complexes and/or at least one of said wound healing promoting agent, selected from the group comprising allantoin, an azulene compound, a compound from the vitamin B series in the form of a liposome preparation.

2. A preparation according to claim 1, wherein at least the greatest part of said agent is encapsulated inside the liposomes.

3. A preparation according to claim 1 or 2,
**characterised in** that the antiseptic agent is povidone iodine.

4. A preparation according to any one of the preceding claims,
**characterised in** that the liposome preparation contains at least one antiseptic and at least one wound healing promoting agent.

5. A preparation according to any one of the preceding claims,
**characterised in** that the liposomes have a substantially uniform size in the range between about 20 and about 20,000 nm, preferably in the range between about 50 and about 4,000 nm, more preferably between 500 and 2,500 nm and especially preferably a uniform size of about 1,000 nm diameter.

6. A preparation according to any one of the preceding claims,
**characterised in** that the liposome preparation releases the agent over an extended time period, preferably an extended time period of several hours duration.

7. A preparation according to claim 6,
**characterised in** that the liposome preparation releases the agent at approximately the same release rate over the release time period.

8. A preparation according to any one of the preceding claims,
**characterised in** that the preparation additionally comprises at least one anaesthetically active agent.

9. A preparation according to any one of the preceding claims,
**characterised in** that the preparation contains additives and adjuvants such as conserving agents and consistency forming additives.

10. A preparation according to any one of claims 1 to 9 in the form of a solution or dispersion comprising liposomes, especially in the form of a pharmaceutical drop preparation.

11. A preparation according to any one of claims 1 to 9 in the form of a hydrophilic or amphiphilic cream, comprising the liposomal agent formulation in a hydrophilic or amphiphilic cream base.

12. A preparation according to any one of claims 1 to 9 in the form of a pharmaceutical O/W or W/O lotion.

13. A preparation according to any one of claims 1 to 9 in the form of a pharmaceutical ointment, containing the liposomal agent or agents in a pharmaceutical ointment base.

14. A preparation according to any one of claims 1 to 9 in the form of a pharmaceutical gel, especially a non-alcoholic hydrogel containing the liposomal agent or agents in a pharmaceutically acceptable hydrogel basis.

15. A preparation according to any one of the preceding claims, in the form of a pharmaceutical eyedrop formulation, which comprises:
a) liposomes comprising a pharmaceutically acceptable liposome membrane forming substance; and
b) a 0.1 to 2% PVP iodine solution (at approximately 10% available iodine in the PVP iodine complex) at least most of which is encapsulated by said liposome membranes,
wherein the liposomes are of substantially uniform size between about 50 and about 4,000 nm, and, in case, the formulation additionally comprises customary additives, adjuvants and auxiliary substances of a pharmaceutical eyedrop formulation.

16. A preparation according to claim 15,
**characterised in** that the liposomes are of substantially uniform size, with diameters at around 1,000 nm.

## Patentansprüche

1. Pharmazeutisches Präparat zur externen Anwendung von antiseptischen Wirkstoffen und/oder Wirkstoffen zur Förderung der Wundheilung,
**dadurch gekennzeichnet, daß** das Präparat wenigstens einen der genannten antiseptischen Wirkstoffe, welcher aus der Gruppe, die Jod und Jodkomplexe umfaßt gewählt wird und/oder wenigstens einen der genannten Wirkstoffe zur Förderung der Wundheilung, welcher aus der Gruppe, die Allantoin, eine Azulenverbindung und eine Verbindung aus der Vitamin B-Serie umfaßt, gewählt wird, in Form eines Liposomenpräparates enthält.

2. Präparat nach Anspruch 1, worin wenigstens der größte Teil des genannten Wirkstoffes innerhalb der Liposomen eingeschlossen ist.

3. Präparat nach Anspruch 1 oder 2;
**dadurch gekennzeichnet, daß** der antiseptische Wirkstoff Povidon-Jod ist.

4. Präparat nach einem der vorhergehenden Ansprüche,
**dadurch gekennzeichnet, daß** das Liposomenpräparat wenigstens einen antiseptischen und wenigstens einen die Wundheilung fördernden Wirkstoff enthält.

5. Präparat nach einem der vorhergehenden Ansprüche,
**dadurch gekennzeichnet, daß** die Liposomen eine im wesentlichen einheitliche Größe im Bereich zwischen ungefähr 20 und ungefähr 20.000 nm, vorzugsweise im Bereich zwischen ungefähr 50 und ungefähr 4.000 nm, mehr bevorzugt zwischen 500 und 2.500 nm und besonders bevorzugt eine einheitliche Größe von ungefähr 1.000 nm im Durchmesser aufweisen.

6. Präparat nach einem der vorhergehenden Ansprüche,
**dadurch gekennzeichnet, daß** das Liposomenpräparat den Wirkstoff über eine ausgedehnte Zeitdauer, vorzugsweise eine ausgedehnte Zeitdauer von mehreren Stunden, freisetzt.

7. Präparat nach Anspruch 6,
**dadurch gekennzeichnet, daß** das Liposomenpräparat den Wirkstoff während der Freisetzungsdauer mit ungefähr der gleichen Freisetzungsrate freisetzt.

8. Präparat nach einem der vorhergehenden Ansprüche,
**dadurch gekennzeichnet, daß** das Präparat zusätzlich wenigstens einen anästhetisch wirksamen Wirkstoff umfaßt.

9. Präparat nach einem der vorhergehenden Ansprüche,
**dadurch gekennzeichnet, daß** das Präparat Zusätze und Adjuvantien, wie Konservierungsmittel und Konsistenzbildner enthält.

10. Präparat nach einem der Ansprüche 1 bis 9 welches in Form einer Lösung oder Dispersion, welche Liposomen umfaßt, insbesondere in der Form eines pharmazeutischen Tröpfchenpräparates vorliegt.

11. Präparat nach einem der Ansprüche 1 bis 9 in Form einer hydrophilen oder amphiphilen Creme, die die liposomale Wirkstofformulierung in einer hydrophilen oder amphiphilen Cremegrundlage umfaßt.

12. Präparat nach einem der Ansprüche 1 bis 9 in Form einer pharmazeutischen O/W oder W/O Lotion.

13. Präparat nach einem der Ansprüche 1 bis 9 in Form einer pharmazeutischen Salbe, die den liposomalen Wirkstoff oder Wirkstoffe in einer pharmazeutischen Salbengrundlage enthält.

14. Präparat nach einem der Ansprüche 1 bis 9 in Form eines pharmazeutischen Gels, insbesondere eines nichtalkoholischen Hydrogels, welches den liposomalen Wirkstoff oder Wirkstoffe in einer pharmazeutisch akzeptablen Hydrogelgrundlage enthält.

15. Präparat nach einem der vorhergehenden Ansprüche, in Form einer pharmazeutischen Augentropfenformulierung, welche umfaßt:
a) Liposomen, die eine pharmazeutisch akzeptable die Liposomenmembran bildende Substanz aufweisen; und
b) eine 0,1 bis 2 %ige PVP-Jodlösung (bei ungefähr 10 % verfügbarem Jod in dem PVP-Jodkomplex) wobei wenigstens der Hauptanteil durch die genannte Liposomenmembranen eingeschlossen ist,
worin die Liposomen eine im wesentlichen einheitliche Größe zwischen ungefähr 50 und ungefähr 4.000 nm aufweisen, und gegebenenfalls die Formulierung zusätzlich übliche Zusätze, Adjuvantien und Hilfsstoffe einer pharmazeutischen Augentropfenformulierung umfaßt.

16. Präparat nach Anspruch 15,
**dadurch gekennzeichnet, daß** die Liposomen im wesentlichen eine einheitliche Größe mit einem Durchmesser um 1.000 nm aufweisen.

## Revendications

1. Préparation pharmaceutique pour l'application externe d'agents antiseptiques et/ou d'agents qui favorisent la cicatrisation de blessures,
caractérisée en ce que la préparation contient au moins un desdits agents antiseptiques sélectionné dans le groupe comprenant l'iode et des complexes iodés, et/ou au moins desdits agents favorisant la cicatrisation de blessures sélectionné dans le groupe comprenant l'allantoïne, un composé azulène, un composé de la série des vitamines B sous la forme d'une préparation liposome.

2. Préparation selon la revendication 1, dans laquelle au moins la majeure partie dudit agent est encapsulée dans les liposomes.

3. Préparation selon la revendication 1 ou 2,
caractérisée en ce que l'agent antiseptique est la povidone iode.

4. Préparation selon l'une quelconque des revendications précédentes,
caractérisée en ce que la préparation liposome contient au moins un antiseptique et au moins un agent favorisant la cicatrisation de blessures.

5. Préparation selon l'une quelconque des revendications précédentes,
caractérisée en ce que les liposomes présentent une taille sensiblement uniforme dans le domaine compris entre environ 20 et environ 20 000 nm, de préférence compris dans le domaine entre environ 50 et environ 4 000 nm, de préférence encore entre 500 et 2 500 nm, et de manière particulièrement préférée une taille uniforme d'environ 1 000 nm de diamètre.

6. Préparation selon l'une quelconque des revendications précédentes,
caractérisée en ce que la préparation liposome libère l'agent sur une longue période de temps, de préférence une longue période de temps d'une durée de plusieurs heures.

7. Préparation selon la revendication 6,
caractérisée en ce que la préparation liposome libère l'agent à approximativement la même vitesse de libération pendant la période de temps de libération.

8. Préparation selon l'une quelconque des revendications précédentes,
caractérisée en ce que la préparation comprend de plus au moins un agent actif anesthésiquement.

9. Préparation selon l'une quelconque des revendications précédentes,
caractérisée en ce que la préparation contient des additifs et des adjuvants tels que des agents conservateurs et des additifs formateurs de consistance.

10. Préparation selon l'une quelconque des revendications 1 à 9, sous la forme d'une solution ou dispersion comprenant des liposomes, particulièrement sous la forme d'une préparation pharmaceutique sous forme de gouttes.

11. Préparation selon l'une quelconque des revendications 1 à 9, sous la forme d'une crème hydrophile ou amphiphile, comprenant la formulation liposomale de l'agent dans une base pour crème hydrophile ou amphiphile.

12. Préparation selon l'une quelconque des revendications 1 à 9, sous la forme d'une lotion pharmaceutique huile/eau ou eau/huile.

13. Préparation selon l'une quelconque des revendications 1 à 9, sous la forme d'un onguent pharmaceutique, contenant l'agent ou les agents liposomal(aux) dans une base pour onguent pharmaceutique.

14. Préparation selon l'une quelconque des revendications 1 à 9, sous la forme d'un gel pharmaceutique, particulièrement un hydrogel non-alcoolique contenant l'agent ou les agents liposomal(aux) dans une base pour hydrogel acceptable pharmaceutiquement.

15. Préparation selon l'une quelconque des revendications précédentes, sous la forme d'une formulation pharmaceutique de gouttes pour les yeux, comprenant :
a) des liposomes comprenant une substance formant des membranes de liposomes acceptable pharmaceutiquement; et
b) une solution d'iode PVP 0,1 à 2 % (à approximativement 10 % d'iode disponible dans le complexe d'iode PVP), dont au moins la majeure partie est encapsulée par lesdites membranes de liposomes,
dans laquelle les liposomes présentent une taille sensiblement uniforme comprise entre environ 50 et environ 4 000 nm et, dans certains cas, la formulation comprend de plus des substances auxiliaires, adjuvants et additifs habituels ou usuels d'une formulation pharmaceutique de gouttes pour les yeux.

16. Préparation selon la revendication 15,
caractérisée en ce que les liposomes présentent une taille sensiblement uniforme, avec des diamètres de l'ordre de 1 000 nm.
